(19) Europäisches Patentamt
European Patent Office
Office européen des brevets

(11) **EP 3 076 935 B1**

(12) **EUROPEAN PATENT SPECIFICATION**

(45) Date of publication and mention
of the grant of the patent:
**15.08.2018 Bulletin 2018/33**

(21) Application number: **14803157.8**

(22) Date of filing: **28.11.2014**

(51) Int Cl.:
*A61Q 3/02* (2006.01)          *A61K 8/34* (2006.01)
*A61K 8/49* (2006.01)          *A61K 8/73* (2006.01)
*A61K 8/87* (2006.01)

(86) International application number:
**PCT/EP2014/075922**

(87) International publication number:
**WO 2015/082337 (11.06.2015 Gazette 2015/23)**

(54) **PHOTO-CROSSLINKABLE VARNISH COMPOSITIONS AS BASE COATING AND APPLICATION METHODS**

LICHTVERNETZBARE LACKZUSAMMENSETZUNGEN ALS GRUNDSCHICHT UND ANWENDUNGSVERFAHREN

COMPOSITIONS DE VERNIS PHOTORETICULABLES COMME REVÊTEMENT DE BASE ET PROCÉDÉS D'APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2013 FR 1362085**

(43) Date of publication of application:
**12.10.2016 Bulletin 2016/41**

(73) Proprietor: **L'OREAL**
**75008 Paris (FR)**

(72) Inventors:
• **KERGOSIEN, Guillaume**
**F-92370 Chaville (FR)**
• **RIACHI, Carl**
**F-75013 Paris (FR)**

(74) Representative: **Miszputen, Laurent**
**L'Oréal**
**Service DIPI**
**9 Rue Pierre Dreyfus**
**92110 Clichy (FR)**

(56) References cited:
**WO-A2-2011/011304          DE-A1-102011 102 661**
**GB-A- 2 496 990          US-A- 5 456 905**

**Description**

The subject of the present invention is novel photo-crosslinkable varnish compositions. Compositions of this type preferably correspond to a base coating applied directly in contact with the nail and/or the false nail or to a top coating applied to a base coat. The subject of the present invention is also methods for applying such compositions to nails and/or false nails, and also the use of said compositions for making up and/or caring for the nails and/or false nails.

The nail varnish compositions can be employed as a varnish base or base coat, as a product for making up the nails, or as a finishing composition, also known as a top coat, to be applied to the product for making up the nails, or else as a product for the cosmetic care of the nails. These compositions can be applied both to natural nails and to false nails.

In the nail varnish field, liquid cosmetic compositions are known which are used by first depositing a coating on the nail, and then by subjecting said coating to the action of light radiation, which causes *in situ* polymerization and/or crosslinking reactions within said coating, resulting in polymeric networks which are usually crosslinked. Such photo-crosslinkable compositions, commonly known as "UV gels", and generally based on crosslinkable compounds of (meth)acrylate monomer type, make it possible to obtain a good wear property of the coating deposited on the nail, and are described, for example, in CA 1 306 954, US 5 456 905, US 7 375 144 and FR 2 823 105.

However, conventional "soak-off" UV gels generally exhibit wear property problems when they are not applied by expert manicurists. Moreover, they generally require a step of roughening the nail aimed at sanding down the nail in order to promote the wear property of the photo-crosslinked composition in film form, which can thus considerably damage the nail. Moreover, the removal of such compositions often proves to be difficult and can require a step of scraping the nail with a metal tool, an electric sander, or an abrasive file, harmful to the integrity of the nail.

Among the patent prior art aimed at overcoming these problems, mention may be made of documents US2011/0081306, US2011/0082228, US2011/0274633 and US2012/0083547.

Mention may be also made of the document DE102011102661 disclosing exemples of photo-crosslinkable compositions for the coating of nails containing an acrylate monomer, an urethane-acrylate oligomer, nitrocellulose and as antioxidant p-hydroxyanisole.

Mention may be also made of the document WO11011304 disclosing a radiation curable gel nail coating composition, comprising:from about 40% to about 60% by weight of di-[hydroxyethyl methacrylic] trirnethylhexyl dicarbamate based on the weight of the nail coating composition; from about 3% to about 5% by weight of at least one methacrylic acid ester based on the weight of the nail coating composition; from about 3% to about 6% by weight by weight hydroxyethyl methacrylate based on the weight of the nail coating composition; from about 3% to about 6% by weight hydroxypropyl methacrylate based on the weight of the nail coating composition; from about 0.1% to about 0.4% by weight of a photoinitiator based on the weight of the nail coating composition; and a nail art paint. The exemples 1 to 4 contain nitrocellulose and citric acid as antioxidant.

The document US5456905 discloses a photoreactive coating for application over and for binding with nail polish comprising nitrocellulose, a photoreactive monomer which may be a polyurethane dimethacrylate, a photoinitiator consisting of benzyl ketal and an inhibitor to polymerisation. The inhibitor may be used with an antoxidant as tris-(4-t-butyl-3-hydroxy-2,6-dimethylbenzyl)-s-triazine-2,4,6-tri-one sold as CYANOX 1790® of the company American Cyanamid Co.

Mention may be also made of the document GB2496990 describing a package comprising (i) a nail coating composition comprising : a radiation curable component comprising an acrylate-terminated oligomer which may be a polyurethane dimethacrylate in combination with at least one acrylate monomer; and a thiol component present in an amount of from about 5% to about 90% w/w of the nal coating composition, wherein the thiol component comprises a compound containing two or more SH groups, and wherein when the thiol component consists of a bis and/or tris functional thiol it is present as at least 15% of the nail coating composition; and (ii) a means for applying the nail coating composition to a nail.

The present invention differs from this prior art through the development of a composition with good gloss, in particular having a good gloss persistence over time.

It also has the advantage of being more stable over time, for example not gelling after two months at 45°C.

Furthermore, some products can exhibit performance problems regarding in particular the quality of the makeup result.

Moreover, the step of removing the prior art compositions conventionally uses tools intended to scrape the surface of the nail so as to remove the photo-crosslinked film of composition previously applied, which are capable of damaging the nails.

Finally, the present invention aims to provide novel photo-crosslinkable compositions which have, after photo-crosslinking of the film, a low content of extractable compounds comprising reactive (meth)crylate functions.

The present invention thus aims to provide novel photo-crosslinkable compositions which do not exhibit at least one of the drawbacks of the compositions mentioned above.

In particular, the present invention aims to provide photo-crosslinkable compositions which can be removed with conventional organic solvents, such as acetone, without requiring a tool which is abrasive for the nails.

In particular, the present invention aims to provide photo-crosslinkable compositions which exhibit a good compromise between wear property and makeup removal compared with the photo-crosslinkable compositions described in the prior art or which exist.

The present invention also aims to provide photo-crosslinkable compositions which allow a quality nail makeup result, in particular in terms of homogeneity of result and, where appropriate, of colour.

The present invention aims to provide, according to a first aspect, photo-crosslinkable compositions which are easy to use, including by the user herself, thus making it possible to save time and money.

The present invention relates to a photo-crosslinkable cosmetic composition, in particular for coating a nail or false nail, and more particularly for making up a nail or false nail, comprising, in a physiologically acceptable medium:

- at least one photo-crosslinkable compound comprising at least one polyurethane di(meth)acrylate compound, more preferentially polyurethane dimethacrylate compound,
- nitrocellulose,
- di(tert-butyl) 4-hydroxytoluene,
- at least one secondary antioxidant chosen from a di(t-butyl) hydroxyphenylamino bisoctylthiotriazine compound.

According to preferred embodiments corresponding to at least one of the abovementioned problems:

- the (poly)urethane di(meth)acrylate compounds, is (are) preferably present in a total content greater than or equal to 65%, relative to the total weight of the solids of the composition, in particular between 70% and 90% by weight, relative to the total weight of the solids of the composition;
- said photo-crosslinkable compound(s) is (are) present in a total content such that said photo-crosslinkable compound(s) represent(s) at least 65% by weight, relative to the total weight relative to the total weight of the (poly)(meth)acrylate compounds;
- the composition also comprises at least one (meth)acrylate monomer;
- the nitrocellulose is present in a total content greater than or equal to 4% by weight, relative to the total weight of the solids of the composition, in particular greater than or equal to 6% by weight, relative to the total weight of the solids of the composition and especially between 8% and 15% by weight, relative to the total weight of the solids of the composition;
- the composition comprises at least one volatile solvent, preferably at least one polar volatile solvent advantageously chosen from the group consisting of $C_3$-$C_6$ esters and ketones and mixtures thereof;
- the volatile solvent(s) is (are) present in a total content greater than or equal to 20% by weight, relative to the total weight of the composition, in particular ranging from 25% to 70%, relative to the total weight of the composition;
- the composition comprises at least one plasticizer;
- the composition comprises at least one photoinitiator, the photoinitiator preferably being chosen from the group consisting of $\alpha$-hydroxy ketones, $\alpha$-amino ketones, aromatic ketones preferably combined with a hydrogen-donating compound, aromatic $\alpha$-diketones and acylphosphine oxides, and mixtures thereof, advantageously from the group consisting of acylphosphine oxides;
- the composition is transparent.

The present invention also relates, according to a second aspect of the invention, to a method for coating the nails and/or false nails, in particular for making up and/or caring for the nails and/or false nails, comprising at least the following steps:

A) application, to a nail or a false nail, of a composition as previously defined, via which a coating consisting of at least one coat of said composition is deposited, and
B) exposure of the coated nail or false nail obtained at the end of step A) to UV or visible light radiation.

The present invention also relates, according to a third aspect of the invention, to a method for coating the nails and/or false nails, in particular for making up and/or caring for the nails and/or false nails, comprising at least the following steps:

A) application, to a nail or a false nail, of a first composition such as a nail varnish or a photo-crosslinkable composition, via which a first coating consisting of at least one coat of said first composition is deposited, this first coating being applied directly in contact with the nail or the false nail,
B) optionally, exposure of the coated nail or false nail obtained at the end of step A) to UV or visible light radiation,
C) application, to the first coating resulting from step A) and B), of a composition as previously defined, distinct from the first composition, via which a second coating consisting of at least one coat of this composition is deposited,
D) exposure of the coated nail or false nail obtained at the end of step C) to UV or visible light radiation.

*Solids*

A composition according to the invention advantageously comprises a solids content of greater than or equal to 30%, in particular greater than or equal to 40%, and advantageously less than or equal to 80%, in particular less than or equal to 70%.

For the purposes of the present invention, the "*solids content*" denotes the content of non-volatile matter.

The solids content (abbreviated as SC) of a composition according to the invention is measured using a "Halogen Moisture Analyzer HR 73" commercial halogen desiccator from Mettler Toledo. The measurement is performed on the basis of the weight loss of a sample dried by halogen heating, and thus represents the percentage of residual matter once the volatile matter has evaporated off.

The measurement protocol is as follows:

About 2 g of the composition, hereinafter the sample, are spread on a metal crucible. The sample is photo-crosslinked under a nitrogen stream (in order to prevent the atmospheric oxygen from inhibiting the crosslinking at the surface of the sample). The metal crucible is then placed in the halogen moisture analyzer mentioned above. The sample is then subjected to a temperature of 105°C until a constant weight is obtained. The wet mass of the sample, corresponding to its initial mass before crosslinking, and the dry mass of the sample, corresponding to its mass after crosslinking and halogen heating, are measured using a precision balance.

The experimental error associated with the measurement is of the order of plus or minus 2%.

The solids content is calculated in the following manner:

Solids content (expressed as weight percentage) = 100 × (dry mass/wet mass).

*Physiologically acceptable medium*

The cosmetic compositions according to the invention comprise a physiologically acceptable medium.

The term "physiologically acceptable medium" is intended to denote a medium that is particularly suitable for applying a composition of the invention to keratin materials.

The physiologically acceptable medium is generally adapted to the nature of the support onto which the composition has to be applied, and also to the appearance under which the composition has to be packaged.

*Photo-crosslinkable compounds*

A composition according to the invention comprises at least one photo-crosslinkable compound.

A composition according to the invention can thus comprise a single photo-crosslinkable compound or a mixture of several photo-crosslinkable compounds.

In the context of the present invention, the term "photo-crosslinkable compounds" denotes organic compounds capable of crosslinking under the action of light radiation, resulting in a crosslinked polymeric network.

The photo-crosslinkable compounds comprise at least one (meth)acrylate function, namely at least one $H_2C=C(R)-C(O)-O-$ function, with R = H or $CH_3$, more preferably R = $CH_3$.

The photo-crosslinkable compound(s) comprise(s) at least one polyurethane **di(meth)acrylate compound**

The "urethane" function is also referred to as "carbamate" function. Preferably, the photo-crosslinkable compound(s) comprise(s) several urethane or carbamate functions.

Thus, the term "poly(meth)acrylate" denotes a compound comprising at least two methacrylate functions, or at least two acrylate functions, or else at least one methacrylate function and at least one acrylate function, preferably at least two methacrylate functions. Preferably, the photo-crosslinkable compound(s) therefore comprise(s) at least one polyurethane dimethacrylate compound comprising a plurality of urethane functions -O-C(O)-NH-, in particular at least two urethane functions, and a plurality of methacrylate functions of formula $H_2C=C(CH_3)-C(O)-O-$, in particular at least two methacrylate functions.

This (these) photo-crosslinkable compound(s) advantageously has (have) a molecular weight greater than 1000 g/mol, in particular between 1000 and 5000 g/mol, better still between 1000 and 3000 g/mol, for example of approximately 1140 g/mol.

The polyurethane di(meth)acrylate compounds, is (are) preferably present in a total content greater than or equal to 65%, relative to the total weight of the solids of the composition, in particular between 70% and 90% by weight, relative to the total weight of the solids of the composition.

The polyurethane di(meth)acrylate compounds, may represent at least 65% by weight, relative to the total weight of the (poly)(meth)acrylate compounds, in particular from 65% to 100% by weight, better still from 80% to 100% by weight, relative to the total weight of the (poly)(meth)acrylate compounds.

### *Film-forming polymer(s)*

The compositions according to the invention also comprise nitrocellulose. Nitrocellulose is a film-forming polymer.

For the purposes of the present invention, the term "film-forming polymer" denotes a polymer that is capable, by itself (i.e. in the absence of an auxiliary film-forming agent or of an external stimulus for example of the UV type), of forming an isolable and in particular continuous and adherent film, on a support, in particular on the nails.

The function of the nitrocellulose is to confer a wear property on the photo-crosslinkable composition and also to promote removal of said composition.

According to one particular preferred embodiment, nitrocellulose is the only film-forming polymer present in a composition in accordance with the invention.

The film-forming polymer(s) may be present in a total content greater than or equal to 4% by weight, relative to the total weight of the solids of the composition, in particular greater than or equal to 6% by weight, relative to the total weight of the solids of the composition and especially between 8% and 15% by weight, relative to the total weight of the solids of the composition.

According to one particular preferred embodiment, the nitrocellulose is present in a total content greater than or equal to 4% by weight, relative to the total weight of the solids of the composition, in particular greater than or equal to 6% by weight, relative to the total weight of the solids of the composition and especially between 8% and 15% by weight, relative to the total weight of the solids of the composition.

According to one particular embodiment, the film-forming polymer(s), in particular the nitrocellulose, and the photo-crosslinkable compound(s), are present in a respective content such that the weight ratio of the film-forming polymer(s), in particular the nitrocellulose, and the photo-crosslinkable compound(s), is preferably greater than or equal to 0.05, preferably less than or equal to 0.20, and preferentially ranges from 0.10 to 0.15.

### *Primary antioxidant(s)*

The compositions according to the invention comprise the primary antioxidant di(tert-butyl) 4-hydroxytoluene.

Such an antioxidant is preferably present in a total content greater than or equal to 0.01% by weight, relative to the total weight of the solids of the composition, in particular greater than or equal to 0.1% by weight, relative to the total weight of the solids of the composition and especially between 0.05% and 1% by weight, relative to the total weight of the solids of the composition.

According to one particular embodiment, the primary antioxidant(s) di(tert-butyl) 4-hydroxytoluene, and the photo-crosslinkable compound(s), are present in a respective content such that the weight ratio of the primary antioxidant di(tert-butyl) 4-hydroxytoluene, and the photo-crosslinkable compound(s), is preferably greater than or equal to 0.0005, preferably less than or equal to 0.1, and preferentially ranges from 0.002 to 0.02.

### *Secondary antioxidant(s)*

The compositions according to the invention comprise at least one secondary antioxidant.

This secondary antioxidant is chosen from a di(t-butyl) hydroxyphenylamino bisoctylthiotriazine compound.

Such an antioxidant is preferably present in a total content greater than or equal to 0.01% by weight, relative to the total weight of the solids of the composition, in particular greater than or equal to 0.05% by weight, relative to the total weight of the solids of the composition and especially between 0.02% and 0.5% by weight, relative to the total weight of the solids of the composition.

According to one particular embodiment, the secondary antioxidant di(t-butyl) hydroxyphenylamino bisoctylthiotriazine, and the photo-crosslinkable compound(s), are present in a respective content such that the weight ratio of the secondary antioxidant di(t-butyl) hydroxyphenylamino bisoctylthiotriazine, and the photo-crosslinkable compound(s), is preferably greater than or equal to 0.0001, preferably less than or equal to 0.02, and preferentially ranges from 0.0002 to 0.005.

### *Volatile solvent(s)*

The compositions according to the invention also advantageously comprise at least one volatile solvent. They can therefore comprise a single volatile solvent or a mixture of several volatile solvents, preferably a mixture of several volatile solvents.

The weight content of volatile solvents is preferably greater than or equal to 20% by weight, relative to the total weight of the composition, in particular ranging from 25% to 70%, relative to the total weight of the composition.

For the purposes of the invention, the term "volatile solvent" is intended to mean a solvent that is capable of evaporating on contact with keratin materials in less than one hour, at ambient temperature and atmospheric pressure.

The volatile solvent(s) of the invention is (are) solvents which are liquid at ambient temperature and which have a non-zero vapour pressure, at ambient temperature and atmospheric pressure, ranging in particular from 50 Pa to 40 000 Pa (0.375 to 300 mmHg), in particular ranging from 100 Pa to 26 664 Pa (0.75 to 200 mmHg) and more particularly ranging from 1000 Pa to 13332 Pa (7.5 to 100 mmHg).

Such solvents aim in particular to fluidize and reduce the solids of the composition.

Preferably, the solvents are chosen from polar solvents.

For the purposes of the present invention, the term "polar solvent" is intended to mean a solvent, or an oil, of which the solubility parameter calculated above its melting point $\delta_a$ is other than 0 $(J/cm^3)^{1/2}$.

The definition and calculation of the solubility parameters in the Hansen three-dimensional solubility space are described in the article by C.M. Hansen: "The three dimensional solubility parameters" J. Paint Technol. 39, 105 (1967).

According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{1/2}$.

The parameters $\delta_p$, $\delta_h$, $\delta_D$ and $\delta_a$ are expressed in $(J/cm^3)^{1/2}$.

In particular, the term "polar" solvent is intended to mean a solvent of which the chemical structure is formed essentially from, or even consists of, carbon and hydrogen atoms, and which comprises at least one highly electronegative heteroatom such as an oxygen, nitrogen, silicon or phosphorus atom.

Preferably, this polar volatile solvent is chosen from the group consisting of $C_3$-$C_6$ esters and ketones and mixtures thereof.

By way of polar volatile solvent, mention may in particular be made of acetone, methyl ethyl ketone, methyl isobutyl ketone, cyclohexanone and alkyl acetates in which the alkyl group comprises from 2 to 5 carbon atoms, such as methyl acetate, ethyl acetate, propyl acetate, n-propyl acetate, isopropyl acetate, n-butyl acetate, isobutyl acetate and tert-butyl acetate.

Preferably, the polar volatile solvent is chosen from the group consisting of ethyl acetate, propyl acetate, such as n-propyl or isopropyl acetate, n-butyl, isobutyl or tert-butyl acetate, isopropanol, and a mixture or mixtures thereof.

According to one preferred embodiment, the solvent is a mixture of butyl acetate, ethyl acetate and propyl acetate.

The butyl acetate, the ethyl acetate and the propyl acetate are preferably present in a respective content ranging respectively from 8% to 15% by weight, from 10% to 20% by weight, and from 8% to 15% by weight, relative to the total weight of the composition.

### Photoinitiator(s)

The compositions according to the invention also advantageously comprise at least one photoinitiator.

They can comprise a single photoinitiator or a mixture of several photoinitiators, preferably a single photoinitiator.

The photoinitiators that can be used according to the present invention are known in the art and are described, for example, in "Les photoinitiateurs dans la reticulation des revêtements" ["Photoinitiators in the crosslinking of coatings"], G. Li Bassi, Double Liaison - Chimie des Peintures, n°361, November 1985, p.34-41; "Applications industrielles de la polymerisation photoinduite" ["Industrial applications of photoinduced polymerization"], Henri Strub, L'Actualité Chimique, February 2000, p.5-13; and "Photopolymères: considerations théoriques et reaction de prise" ["Photopolymers: theoretical considerations and setting reaction"], Marc, J.M. Abadie, Double Liaison - Chimie des Peintures, n°435-436, 1992, p.28-34.

These photoinitiators encompass:

- α-hydroxy ketones, sold, for example, under the names Darocur® 1173 and 4265, Irgacure® 184, 2959, and 500 by the company BASF, and Additol® CPK by the company Cytec,

- α-amino ketones, sold, for example, under the names Irgacure® 907 and 369 by the company BASF,
- aromatic ketones, sold, for example, under the name Esacure® TZT by Lamberti. Mention may also be made of the thioxanthones sold, for example, under the name Esacure® ITX by Lamberti, and quinones. These aromatic ketones usually require the presence of a hydrogen-donating compound such as tertiary amines and in particular alkanolamines. Mention may in particular be made of the tertiary amine Esacure® EDB sold by the company Lamberti;
- α-dicarbonyl derivatives, the most common representative of which is benzyl dimethyl ketal, sold under the name Irgacure® 651 by BASF. Other commercial products are sold by the company Lamberti under the name Esacure® KB1, and
- acylphosphine oxides such as, for example, the bis-acylphosphine oxides (BAPOs) sold, for example, under the names Irgacure® 819, 1700 and 1800 and Darocur® 4265, Lucirin® TPO and Lucirin® TPO-L by the company BASF.

Preferably, the photoinitiator is chosen from the group consisting of α-hydroxy ketones, α-amino ketones, aromatic ketones preferably combined with a hydrogen-donating compound, aromatic α-diketones and acylphosphine oxides, and mixtures thereof.

An acylphosphine oxide is preferably used in the photo-crosslinkable composition of the invention.

By way of photoinitiator, mention may be made of Lucirin® TPO-L (BASF).

The total content of the photoinitiator(s) depends on a large number of factors such as, for example, the reactivity of the various components of the mixture, the presence of a colouring agent or colouring agents, the intensity of the light source or the exposure time.

In order to obtain the desired properties, the photoinitiator(s) is (are) preferably present in a total content greater than or equal to 0.1% by weight, relative to the total weight of the photo-crosslinkable composition, preferably ranging from 0.2% to 5% by weight, relative to the total weight of the photo-crosslinkable composition.

**Plasticizer(s)**

The photo-crosslinkable compositions of the invention may also comprise one or more plasticizer(s).

According to one embodiment, these compositions comprise less than 15% by weight of plasticizer(s), relative to the total weight of said composition.

Preferably, the weight content of plasticizer ranges from 0% to 15%, preferentially from 1% to 10%, and more preferentially from 2% to 5% by weight, relative to the total weight of said composition.

By way of plasticizers, mention may in particular be made of the usual plasticizers, such as glycols and derivatives thereof, such as diethylene glycol ethyl ether, diethylene glycol methyl ether, diethylene glycol butyl ether or else diethylene glycol hexyl ether, ethylene glycol ethyl ether, ethylene glycol methyl ether, ethylene glycol butyl ether or ethylene glycol hexyl ether; glycol esters, propylene glycol derivatives and in particular propylene glycol phenyl ether, propylene glycol diacetate, dipropylene glycol butyl ether, tripropylene glycol butyl ether, propylene glycol methyl ether, dipropylene glycol ethyl ether, tripropylene glycol methyl ether and diethylene glycol methyl ether or propylene glycol butyl ether; acid esters, especially carboxylic acid esters, such as citrates, especially triethyl citrate, tributyl citrate, triethyl acetylcitrate, tributyl acetylcitrate or 2-triethylhexyl acetylcitrate; phthalates, in particular dimethoxyethyl phthalate; phosphates, in particular tricresyl phosphate, tributyl phosphate, triphenyl phosphate or tributoxyethyl phosphate; tartrates, especially dibutyl tartrate; adipates; carbonates; sebacates; benzyl benzoate, butyl acetylricinoleate, glyceryl acetylricinoleate, butyl glycolate, camphor, glyceryl triacetate or N-ethyl-o,p-toluenesulfonamide; oxyethylenated derivatives such as oxyethylenated oils, in particular vegetable oils, such as castor oil, silicone oils, hydrocarbon-based oils, and mixtures thereof.

According to one particular embodiment, a composition in accordance with the present invention comprises at least one citrate compound, in particular tributyl acetylcitrate.

According to one particular embodiment, a composition in accordance with the present invention comprises at least one N-ethyl-o,p-toluenesulfonamide compound.

According to a more particular embodiment, a composition in accordance with the present invention comprises at least one citrate compound, in particular tributyl acetylcitrate, and at least one N-ethyl-o,p-toluenesulfonamide compound.

***Other constituents***

According to one particular embodiment, a composition according to the invention may comprise at least one (meth)acrylate monomer, such as a tetrahydrofurfuryl methacrylate compound. In particular, this (these) (meth)acrylate monomer(s) may be present in order to improve the wear property and the mechanical property. This (these) monomer(s) is (are) preferably present in a content less than or equal to 10% by weight, relative to the total weight of the solids of the composition, better still in a content less than or equal to 5% by weight, relative to the total weight of the solids of the composition.

The compositions according to the invention can also contain adjuvants, or additives, chosen in particular from colouring agents such as pigments, coalescers, preservatives, thickeners, fragrances, cosmetic nail care active agents, spreading agents, antifoams and dispersants.

Needless to say, those skilled in the art will take care to choose these optional adjuvants and additives such that the advantageous properties of the composition according to the invention are not, or are virtually not, adversely affected by the envisaged addition.

When the composition comprises colouring agents, the absorption spectrum of the colouring agents used should in particular be adapted to that of the photoinitiators, or conversely the absorption spectrum of the photoinitiators to that of the colouring agents used, in order to avoid these two types of compounds absorbing light at the same wavelengths. This is because the absorption of light by the colouring agents would render almost totally ineffective the photoinitiators present beyond a certain depth of the coating.

Preferably, the composition of the invention is transparent.

As used herein, the term "transparent" means that the composition has a HAZEBYK index of less than 5 as measured with a KYKHAZEGLOSS brilliance meter.

According to one embodiment, the composition of the invention also comprises a colouring agent chosen from the group consisting of soluble dyes, pigments, nacres and glitter flakes.

The colouring agent(s) can be present in a total content greater than or equal to 0.1% by weight, relative to the total weight of the coat, preferably ranging from 0.1% to 5%, advantageously from 0.2% to 1% by weight, relative to the total weight of the composition.

The term "soluble dyes" should be understood as meaning organic, inorganic or organometallic compounds which are soluble in the composition of the invention and intended to colour said composition.

The dyes are, for example, Sudan red, DC red 17, DC green 6, β-carotene, soybean oil, Sudan brown, DC yellow 11, DC violet 2, DC orange 5 and quinoline yellow.

The term "pigments" should be understood as meaning white or coloured and inorganic or organic particles of any shape which are insoluble in the composition of the invention and which are intended to colour said composition.

The term "nacres" should be understood as meaning iridescent particles of any shape, in particular produced by certain molluscs in their shell, or else synthesized.

The pigments may be white or coloured, and inorganic and/or organic. Among the inorganic pigments that may be mentioned are titanium dioxide, optionally surface-treated, zirconium oxide or cerium oxide, and also zinc oxide, iron (black, yellow or red) oxide or chromium oxide, manganese violet, ultramarine blue, chromium hydrate and ferric blue, and metal powders, for instance aluminium powder and copper powder.

Among the organic pigments that may be mentioned are carbon black, pigments of D & C type and lakes based on cochineal carmine or on barium, strontium, calcium or aluminium.

Mention may also be made of effect pigments, such as particles comprising an organic or inorganic, natural or synthetic substrate, for example glass, acrylic resins, polyester, polyurethane, polyethylene terephthalate, ceramics or aluminas, which may or may not be covered with metal substances, such as aluminium, gold, copper or bronze, or with metal oxides, such as titanium dioxide, iron oxide or chromium oxide, or with inorganic or organic pigments, and mixtures thereof.

The nacreous pigments can be chosen from white nacreous pigments, such as mica covered with titanium or with bismuth oxychloride, coloured nacreous pigments, such as titanium mica covered with iron oxides, titanium mica covered with in particular ferric blue or with chromium oxide, or titanium mica covered with an organic pigment of the abovementioned type, and nacreous pigments based on bismuth oxychloride.

Use may also be made of pigments with goniochromatic properties, in particular liquid crystal or multilayer pigments.

Optical brighteners or fibres optionally coated with optical brighteners can also be used.

The compositions according to the invention may also comprise one or more fillers, in particular in a content ranging from 0.01% to 50% by weight, relative to the total weight of the composition, and preferably ranging from 0.01% to 30% by weight.

The term "fillers" should be understood as meaning colourless or white, inorganic or synthetic particles of any shape, which are insoluble in the medium of the composition, irrespective of the temperature at which the composition is manufactured. These fillers serve in particular to modify the rheology or the texture of the composition.

The fillers may be inorganic or organic and of any shape, platelet-shaped, spherical or oblong, irrespective of the crystallographic form (for example lamellar, cubic, hexagonal, orthorhombic, etc.). Mention may be made of talc, mica, silica, kaolin, polyamide (Nylon®) powder (Orgasol® from Atochem), poly-p-alanine powder and polyethylene powder, powders of tetrafluoroethylene polymers (Teflon®), lauroyllysine, starch, boron nitride, hollow polymeric microspheres such as those of polyvinylidene chloride/acrylonitrile, for instance Expancel® (Nobel Industrie) or of acrylic acid copolymers (Polytrap® from the company Dow Corning) and silicone resin microbeads (for example Tospearls® from Toshiba), elastomeric polyorganosiloxane particles, precipitated calcium carbonate, magnesium carbonate, magnesium hydrogen carbonate, hydroxyapatite, hollow silica microspheres (Silica Beads® from Maprecos), glass or ceramic microcapsules, and metal soaps derived from organic carboxylic acids containing from 8 to 22 carbon atoms and preferably from 12 to 18 carbon atoms, for example zinc stearate, magnesium stearate or lithium stearate, zinc laurate or magnesium myristate.

*Uses*

According to one embodiment, the compositions of the invention are intended to be applied to the nails and/or false nails, preferably for making up and/or caring for the nails and/or false nails, more preferentially for making up the nails and/or false nails.

According to one preferred embodiment, the compositions of the invention are intended to be applied to the nails and/or false nails as a top coat intended to directly coat the nail or a false nail, or to coat a coloured nail varnish previously applied to the nail or the false nail.

According to one particular embodiment, a method for coating the nails and/or false nails, in particular for making up and/or caring for the nails and/or false nails, comprises at least the following steps:

A) application, to a nail or a false nail, of a composition according to the invention, via which a coating consisting of at least one coat of said composition is deposited, and
B) exposure of the coated nail or false nail obtained at the end of step A) to UV or visible light radiation.

According to one particular embodiment, a method for coating the nails and/or false nails, in particular for making up and/or caring for the nails and/or false nails, comprises at least the following steps:

A) application, to a nail or a false nail, of a first composition, preferably a nail varnish or a photo-crosslinkable composition, via which a first coating consisting of at least one coat of said first composition is deposited, this first coating being applied directly in contact with the nail or the false nail,
B) optionally, exposure of the coated nail or false nail obtained at the end of step A) to UV or visible light radiation,
C) application, to the first coating resulting from step A) and B), of a composition according to the invention, distinct from the first composition, via which a second coating consisting of at least one coat of this composition is deposited,
D) exposure of the coated nail or false nail obtained at the end of step C) to UV or visible light radiation.

A subject of the present invention is also a kit comprising:

- a photo-crosslinkable cosmetic composition according to the invention,
- an abrasive material having a particle size of greater than or equal to 200, preferably less than 300, advantageously between 220 and 280, and
- an LED lamp or a UV lamp.

A subject of the present invention is also a method for coating a nail and/or false nail, comprising the following steps:

i) rubbing the surface of a nail or of the false nail with an abrasive material having a particle size of greater than or equal to 200, preferably less than 300, advantageously between 220 and 280,
ii) applying a photo-crosslinkable cosmetic composition according to the invention to the surface of the nail or of false nails which has been rubbed following step i), wherein a coat consisting of at least one coat of said composition according to the invention is deposited,
iii) exposing the coated nail or false nails obtained following step ii) to an LED lamp or a UV lamp, such that the photo-crosslinking is carried out so as to obtain a crosslinked coat.

The rubbing step is carried out for less than 10 seconds, preferably less than 5 seconds, for example for approximately 3 seconds.

The weight percentages given in this application can be categorized as the percentage by weight of dry matter of the compounds used, unless otherwise expressly mentioned.

The invention will be better understood on reading the following description, given solely by way of example.

## EXAMPLE

According to a first exemplary embodiment, a conventional nail varnish of the Essie brand is pre-applied to a nail.

A top composition in accordance with the invention, or top coat, is then prepared with a view to applying it to said nail varnish.

Composition according to the invention:

| Ingredients of the top composition according to the invention | % Content |
|---|---|
| Nitrocellulose containing 30% of isopropyl alcohol (viscosity: E22 - 1/2s) (IDYL E35 TX IPA 30% from BERGERAC - SNPE) | 8.76 |
| Urethane dimethacrylate (EXOTHANE 10 - ESSTECH, Inc.) | 38.52 |
| Urethane dimethacrylate (EXOTHANE 32 - ESSTECH, Inc.) | 9.63 |
| Tributyl acetyl citrate | 1.94 |
| N-ethyl-o,p-toluenesulfonamide | 1.55 |
| Ethyl acetate | 13.53 |
| Propyl acetate | 11.82 |
| Butyl acetate | 11.82 |
| Photoinitiator ethyl (2,4,6-trimethylbenzoyl) phenylphosphinate (Lucirin TPO-L - BASF) | 2 |
| BHT (DI-(TERT-BUTYL)-4-HYDROXYTOLUENE - NIPANOX BHT from CLARIANT) | 0.33 |
| DI-(T-BUTYL) HYROXYPHENYLAMINO BISOCTYLTHIOTRIAZINE (TINOGARD MD1 from CIBA-BASF) | 0.1 |

The ingredients of the composition are introduced into an opaque flask and stirred away from light with a laboratory mixer of the Rayneri brand until a homogeneous mixture is obtained. A sheet of aluminium will have been placed over the container beforehand in order to prevent evaporation of the solvents.

The top composition described above is then applied to the varnish in the form of one or more coats, in this case a single coat. After the application of this coat, the nail is placed under a 36 W UV lamp for 3 minutes in order to crosslink the composition so as to form a top coating in the form of a film.

After having crosslinked the final coat, the surface is cleaned with cotton wool soaked in isopropanol in order to remove the tacky coat.

Comparative composition:

| Ingredients of the top composition according to the invention | % Content |
|---|---|
| Nitrocellulose containing 30% of isopropyl alcohol (viscosity: E22 - 1/2s) (IDYL E35 TX IPA 30% from BERGERAC - SNPE) | 8.76 |
| Urethane dimethacrylate (EXOTHANE 10 - ESSTECH, Inc.) | 38.52 |
| Urethane dimethacrylate (EXOTHANE 32 - ESSTECH, Inc.) | 9.63 |
| Tributyl acetyl citrate | 1.94 |
| N-ethyl-o,p-toluenesulfonamide | 1.55 |
| Ethyl acetate | 13.63 |
| Propyl acetate | 11.82 |
| Butyl acetate | 11.82 |
| Photoinitiator ethyl (2,4,6-trimethylbenzoyl) phenylphosphinate (Lucirin TPO-L - BASF) | 2 |
| BHT (DI-(TERT-BUTYL)-4-HYDROXYTOLUENE - NIPANOX BHT from CLARIANT) | 0.33 |

As previously, a conventional nail varnish of the Essie brand is pre-applied to a nail.

The comparative top composition above is then applied to the varnish in the form of one or more coats, in this case a single coat. After the application of this coat, the nail is placed under a 36 W UV lamp for 3 minutes in order to crosslink the composition so as to form a top coating in the form of a film.

After having crosslinked the final coat, the surface is cleaned with cotton wool soaked in isopropanol in order to remove the tacky coat.

In the two embodiments according to the invention evaluated, each comprising a base coating comprising a photo-crosslinkable composition in accordance with invention, a varnish exhibiting a good wear property on the nail is thus obtained. Such a varnish is glossy and exhibits a good wear property of the gloss on the nail. To do this, the wear property was assessed by simple observation with the naked eye after 14 days following its application. This wear performance is produced with only a very slight roughening of the nail before application of said compositions, making it possible to avoid the conventional invasive method of attaching a photo-crosslinkable composition to the nails by sanding the surface of the nail, while at the same time preserving performance levels equivalent to or even better than products currently on the market.

Furthermore, the ingredients used in the compositions according to the invention make it possible to have, after photo-crosslinking of the film, an extremely low content of extractable compounds comprising reactive (meth)acrylate functions with potentially sensitizing effects.

The varnish can then be completely removed after having been in contact with acetone for 15 minutes, this time again therefore without a conventional invasive method using a metal tool, an electric sander, or an abrasive file by rubbing against the surface of the made-up nail in order to remove the composition.

Finally, the composition according to the invention comprising a second antioxidant exhibits better stability than the comparative composition not comprising a second antioxidant.

Thus, the comparative composition gels after two months at 45°C, whereas the composition according to the invention remains stable.

Throughout the application, the wording "comprising one" or "including one" means "comprising at least one" or "including at least one", unless otherwise specified.

## Claims

1. Photo-crosslinkable cosmetic composition, in particular for coating a nail or false nail, and more particularly for making up a nail or false nail, comprising, in a physiologically acceptable medium:

   - at least one photo-crosslinkable compound comprising at least one polyurethane di(meth)acrylate compound, more preferentially polyurethane dimethacrylate compound,
   - nitrocellulose, **di(tert-butyl) 4-hydroxytoluene,**
   - at least one secondary antioxidant chosen from a di(t-butyl) hydroxyphenylamino bisoctylthiotriazine compound.

2. Composition according to Claim 1, in which the the (poly)urethane di(meth)acrylate compounds, is (are) preferably present in a total content greater than or equal to 65%, relative to the total weight of the solids of the composition, in particular between 70% and 90% by weight, relative to the total weight of the solids of the composition.

3. Composition according to Claim 1 or 2, in which said photo-crosslinkable compound(s) is (are) present in a total content such that said photo-crosslinkable compound(s) represent(s) at least 65% by weight, relative to the total weight of the (poly)(meth)acrylate compounds.

4. Composition according to Claim 1, 2 or 3, also comprising at least one (meth)acrylate monomer.

5. Composition according to any one of the preceding claims, in which the nitrocellulose is present in a total content greater than or equal to 4% by weight, relative to the total weight of the solids of the composition, in particular greater than or equal to 6% by weight, relative to the total weight of the solids of the composition and especially between 8% and 15% by weight, relative to the total weight of the solids of the composition.

6. Composition according to any one of the preceding claims, comprising at least one volatile solvent, preferably at least one polar volatile solvent advantageously chosen from the group consisting of $C_3$-$C_6$ esters and ketones and mixtures thereof.

7. Composition according to any one of the preceding claims, in which the volatile solvent(s) is (are) present in a total content greater than or equal to 20% by weight, relative to the total weight of the composition, in particular ranging from 25% to 70%, relative to the total weight of the composition.

8. Composition according to any one of the preceding claims, comprising at least one plasticizer.

9. Composition according to any one of the preceding claims, comprising at least one photoinitiator, the photoinitiator preferably being chosen from the group consisting of $\alpha$-hydroxy ketones, $\alpha$-amino ketones, aromatic ketones preferably combined with a hydrogen-donating compound, aromatic $\alpha$-diketones and acylphosphine oxides, and mixtures thereof, advantageously from the group consisting of acylphosphine oxides.

10. Method for coating the nails and/or false nails, in particular for making up and/or caring for the nails and/or false nails, comprising at least the following steps:

A) application, to a nail or a false nail, of a composition according to any one of Claims 1 to 9, via which a coating consisting of at least one coat of said composition is deposited, and
B) exposure of the coated nail or false nail obtained at the end of step A) to UV or visible light radiation.

11. Method for coating the nails and/or false nails, in particular for making up and/or caring for the nails and/or false nails, comprising at least the following steps:

A) application, to a nail or a false nail, of a first composition, such as a nail varnish, via which a first coating consisting of at least one coat of said composition is deposited, this first coating being applied directly in contact with the nail or the false nail,
B) optionally, exposure of the coated nail or false nail obtained at the end of step A) to UV or visible light radiation,
C) application, to the first coating resulting from step A) and B), of a composition according to any one of Claims 1 to 9, distinct from the first composition, via which a second coating consisting of at least one coat of this composition is deposited,
D) exposure of the coated nail or false nail obtained at the end of step C) to UV or visible light radiation.

## Patentansprüche

1. Photovernetzbare kosmetische Zusammensetzung, insbesondere zum Beschichten eines Nagels oder eines künstlichen Nagels und insbesondere zum Zurechtmachen eines Nagels oder eines künstlichen Nagels, umfassend in einem physiologisch unbedenklichen Medium:

- mindestens eine photovernetzbare Verbindung, die mindestens eine Polyurethandi(meth)acrylat-Verbindung, weiter bevorzugt Polyurethandimethacrylat-Verbindung, umfasst,
- Nitrocellulose,
- Di(tert-butyl)-4-hydroxytoluol
- mindestens ein sekundäres Antioxidans, das aus einer Di(t-butyl)hydroxyphenylaminobisoctylthiotriazin-Verbindung ausgewählt ist.

2. Zusammensetzung nach Anspruch 1, wobei die Polyurethandi(meth)acrylat-Verbindungen in einem Gesamtgehalt größer oder gleich 65 Gew.-%, bezogen auf das Gesamtgewicht der Feststoffe der Zusammensetzung, insbesondere zwischen 70 und 90 Gew.-%, bezogen auf das Gesamtgewicht der Feststoffe der Zusammensetzung, vorliegt bzw. vorliegen.

3. Zusammensetzung nach Anspruch 1 oder 2, wobei die photovernetzbare Verbindung bzw. die photovernetzbaren Verbindungen in einem solchen Gesamtgehalt vorliegt bzw. vorliegen, dass die photovernetzbare Verbindung bzw. die photovernetzbaren Verbindungen mindestens 65 Gew.-%, bezogen auf das Gesamtgewicht der (Poly)(meth)-acrylat-Verbindungen, ausmacht bzw. ausmachen.

4. Zusammensetzung nach Anspruch 1, 2 oder 3, außerdem umfassend mindestens ein (Meth)acrylat-Monomer.

5. Zusammensetzung nach einem der vorhergehenden Ansprüche, wobei die Nitrocellulose in einem Gesamtgehalt größer oder gleich 4 Gew.-%, bezogen auf das Gesamtgewicht der Feststoffe der Zusammensetzung, insbesondere größer oder gleich 6 Gew.-%, bezogen auf das Gesamtgewicht der Feststoffe in der Zusammensetzung, und speziell zwischen 8 und 15 Gew.-%, bezogen auf das Gesamtgewicht der Feststoffe der Zusammensetzung, vorliegt.

6. Zusammensetzung nach einem der vorhergehenden Ansprüche, umfassend mindestens ein flüchtiges Lösungsmittel, vorzugsweise mindestens ein polares flüchtiges Lösungsmittel, das vorteilhafterweise aus der Gruppe bestehend aus $C_3$-$C_6$-Estern und -Ketonen und Mischungen davon ausgewählt ist.

7. Zusammensetzung nach einem der vorhergehenden Ansprüche, wobei das flüchtige Lösungsmittel bzw. die flüchtigen Lösungsmittel in einem Gesamtgehalt größer oder gleich 20 Gew.-%, bezogen auf das Gesamtgewicht der Zusammensetzung, insbesondere im Bereich von 25 % bis 70 %, bezogen auf das Gesamtgewicht der Zusammensetzung, vorliegt bzw. vorliegen.

8. Zusammensetzung nach einem der vorhergehenden Ansprüche, umfassend mindestens einen Weichmacher.

9. Zusammensetzung nach einem der vorhergehenden Ansprüche, umfassend mindestens einen Photoinitiator, wobei der Photoinitiator vorzugsweise aus der Gruppe bestehend aus α-Hydroxyketonen, α-Aminoketonen, aromatischen Ketonen, vorzugsweise in Kombination mit einem Wasserstoffdonor, aromatischen α-Diketonen und Acylphosphinoxiden und Mischungen davon, vorteilhafterweise aus der Gruppe bestehend aus Acylphosphinoxiden, ausgewählt ist.

10. Verfahren zum Beschichten der Nägel und/oder künstlichen Nägel, insbesondere zum Zurechtmachen und/oder Pflegen der Nägel und/oder künstlichen Nägel, das mindestens die folgenden Schritte umfasst:

A) Aufbringen einer Zusammensetzung nach einem der Ansprüche 1 bis 9 auf einen Nagel oder einen künstlichen Nagel, wodurch eine Beschichtung, die aus mindestens einer Schicht der photovernetzbaren Zusammensetzung besteht, abgeschieden wird, und
B) Einwirkenlassen von UV- oder sichtbarer Lichtstrahlung auf den am Ende von Schritt A) erhaltenen Nagel oder künstlichen Nagel.

11. Verfahren zum Beschichten der Nägel und/oder künstlichen Nägel, insbesondere zum Zurechtmachen und/oder Pflegen der Nägel und/oder künstlichen Nägel, das mindestens die folgenden Schritte umfasst:

A) Aufbringen einer ersten Zusammensetzung, wie eines Nagellacks, auf einen Nagel oder einen künstlichen Nagel, wodurch eine erste Beschichtung, die aus mindestens einer Schicht der photovernetzbaren Zusammensetzung besteht, abgeschieden wird, wobei diese erste Beschichtung direkt in Kontakt mit dem Nagel oder dem künstlichen Nagel aufgebracht wird,
B) gegebenenfalls Einwirkenlassen von UV- oder sichtbarer Lichtstrahlung auf den am Ende von Schritt A) erhaltenen Nagel oder künstlichen Nagel,
C) Aufbringen einer zweiten Zusammensetzung nach einem der Ansprüche 1 bis 9, die von der ersten Zusammensetzung verschieden ist, auf die aus Schritt A) und B) resultierende erste Beschichtung, wodurch eine zweite Beschichtung, die aus mindestens einer Schicht der zweiten Zusammensetzung besteht, abgeschieden wird,
D) Einwirkenlassen von UV- oder sichtbarer Lichtstrahlung auf den am Ende von Schritt C) erhaltenen Nagel oder künstlichen Nagel.

**Revendications**

1. Composition cosmétique photoréticulable, en particulier destinée au revêtement d'un ongle ou faux-ongle, et plus particulièrement destinée au maquillage d'un ongle ou faux-ongle, comprenant, dans un milieu physiologiquement acceptable :

- au moins un composé photoréticulable comprenant au moins un composé polyuréthane di(méth)acrylate, plus préférentiellement un composé polyuréthane diméthacrylate,
- de la nitrocellulose,
- du ditertiobutyl 4-hydroxytoluène,
- au moins un agent anti-oxydant secondaire, choisi parmi un composé di-t-butyl hydroxyphénylamino bisoctylthiotriazine.

2. Composition selon la revendication 1, dans laquelle les composés (poly)uréthane di(méth)acrylates, est/sont de préférence présent(s) à une teneur totale supérieure ou égale à 65 % par rapport au poids total de l'extrait sec de la composition, en particulier compris entre 70 et 90 % en poids, par rapport au poids total de l'extrait sec de la composition.

3. Composition selon la revendication 1 ou 2, dans laquelle ledit/lesdits composé(s) photoréticulable(s) est/sont présent(s) à une teneur totale telle que ledit/lesdits composé(s) photoréticulable(s) représente/représentent au moins 65 % en poids, par rapport au poids total des composés (poly)(méth)acrylate.

4. Composition selon la revendication 1, 2 ou 3, comprenant en outre au moins un monomère (méth)acrylate.

5. Composition selon l'une quelconque des revendications précédentes, dans laquelle la nitrocellulose est présente à une teneur totale supérieure ou égale à 4 % en poids, par rapport au poids total de l'extrait sec de la composition, en particulier supérieure ou égale à 6 % en poids, par rapport au poids total de l'extrait sec de la composition, notamment compris entre 8 et 15 % en poids, par rapport au poids total de l'extrait sec de la composition.

6. Composition selon l'une quelconque des revendications précédentes, comprenant au moins un solvant volatil, de préférence au moins un solvant volatil polaire avantageusement choisi dans le groupe constitué des cétones et des esters en $C_3$-$C_6$ et leurs mélanges.

7. Composition selon l'une quelconque des revendications précédentes, dans laquelle le/les solvants volatil(s) est/sont présent(s) à une teneur totale supérieure ou égale à 20 % en poids par rapport au poids total de la composition, en particulier allant de 25 à 70 % par rapport au poids total de la composition.

8. Composition selon l'une quelconque des revendications précédentes, comprenant au moins un agent plastifiant.

9. Composition selon l'une quelconque des revendications précédentes, comprenant au moins un photoamorceur, le photoamorceur étant de préférence choisi dans le groupe constitué des $\alpha$-hydroxycétones, des $\alpha$-aminocétones, des cétones aromatiques de préférence associées à un composé donneur d'hydrogène, des $\alpha$-dicétones aromatiques et des oxydes d'acylphosphine, et de leurs mélanges, avantageusement dans le groupe constitué des oxydes d'acylphosphine.

10. Procédé de revêtement des ongles et/ou des faux-ongles, en particulier de maquillage et/ou de soin des ongles et/ou des faux-ongles, comprenant au moins les étapes suivantes :

A) application, sur un ongle ou un faux-ongle, d'une composition selon l'une quelconque des revendications 1 à 9, par laquelle on dépose un revêtement constitué d'au moins une couche de ladite composition, et
B) exposition de l'ongle ou du faux-ongle revêtu obtenu à l'issue de l'étape A) à un rayonnement UV ou de lumière visible.

11. Procédé de revêtement des ongles et/ou des faux-ongles, en particulier de maquillage et/ou de soin des ongles et/ou des faux-ongles, comprenant au moins les étapes suivantes :

A) application, sur un ongle ou un faux-ongle, d'une première composition, telle qu'un vernis à ongle, par laquelle on dépose un premier revêtement constitué d'au moins une couche de ladite composition, ce premier revêtement étant appliqué directement au contact de l'ongle ou du faux-ongle,
B) éventuellement exposition de l'ongle ou du faux-ongle revêtu obtenu à l'issue de l'étape A) à un rayonnement UV ou de lumière visible,
C) application, sur le premier revêtement issu des étapes A) et B), d'une composition selon l'une quelconque des revendications 1 à 9, distincte de la première composition, par laquelle on dépose un deuxième revêtement constitué d'au moins une couche de cette composition,
D) exposition de l'ongle ou du faux-ongle revêtu obtenu à l'issue de l'étape C) à un rayonnement UV ou de lumière visible.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 1306954
- US 5456905 A
- US 7375144 B
- FR 2823105
- US 20110081306 A
- US 20110082228 A
- US 20110274633 A
- US 20120083547 A
- DE 102011102661
- WO 11011304 A
- GB 2496990 A

**Non-patent literature cited in the description**

- **C.M. HANSEN.** The three dimensional solubility parameters. *J. Paint Technol.,* 1967, vol. 39, 105
- **G. LI BASSI.** Les photoinitiateurs dans la reticulation des revêtements'' [''Photoinitiators in the crosslinking of coatings. *Double Liaison - Chimie des Peintures,* November 1985, 34-41
- **HENRI STRUB.** Applications industrielles de la polymerisation photoinduite'' [''Industrial applications of photoinduced polymerization. *L'Actualité Chimique,* February 2000, 5-13
- **MARC, J.M. ABADIE.** Photopolymères: considerations théoriques et reaction de prise'' [''Photopolymers: theoretical considerations and setting reaction. *Double Liaison - Chimie des Peintures,* 1992, 28-34